# EUROPEAN PATENT SPECIFICATION

(11) **EP 2 016 937 B1**
(45) Date of publication and mention of the grant of the patent: **21.01.2015**
(21) Application number: 06743460.5
(22) Date of filing: 28.04.2006
(51) Int. Cl.: A61K 9/127, A61K 9/00, A61K 31/07, A61K 31/375, A61K 33/00, A61K 33/06, A61K 33/14, A61K 38/18, A61K 38/38, A61K 38/40, A61K 38/47, A61K 47/22, A61K 38/04, A61K 38/17, A61K 38/30, A61K 38/57

(54) **FORMULATION OF LIPOSOMAL VESICLES IN AQUEOUS SOLUTIONS WITH TEAR FILM CHARACTERISTICS**
FORMULIERUNGEN LIPOSOMALER VESIKEL IN WÄSSRIGEN LÖSUNGEN MIT TRÄNENFILM-EIGENSCHAFTEN
FORMULATION DE VÉSICULES LIPOSOMALES DANS DES SOLUTIONS AQUEUSES PRÉSENTANT DES CARACTÉRISTIQUES DE FILM LACRYMAL

(30) Priority: 27.04.2006 ES 200601078
(43) Date of publication of application: 21.01.2009
(73) Proprietor: UNIVERSIDAD COMPLUTENSE DE MADRID, E-28040 Madrid (ES)
(72) Inventor: MOLINA MARTINEZ, Irene Teresa, 28040 Madrid (ES); VICARIO DE LA TORRE, Marta, 28040 Madrid (ES); BENITEZ DEL CASTILLO, Jose Manuel, 28040 Madrid (ES); VICO RICO, Eva, 28040 Madrid (ES); HERRERO VANREL, Rocío, 28040 Madrid (ES)
(74) Representative: Carpintero Lopez, Francisco
(86) International application number: PCT/ES2006/000208
(87) International publication number: WO 2007/125134

(56) References cited:
- WO-A1-00/51619
- WO-A1-90/11781
- WO-A1-98/43616
- WO-A2-2005/084635
- US-A- 5 064 655
- US-A1- 2005 202 097

## Description

### OBJECT OF THE INVENTION

The present invention relates to the formulation of liposomal vesicles in aqueous solutions with tear film characteristics as defined by the claims. The present invention describes a formulation of liposomes in aqueous vehicles which contain mucin or substances similar to mucin, mucomimetic substances or polymers with mucoadhesive properties which, at the temperature of the corneal surface, have characteristics similar to the precorneal film of the human eye. Said preparation can be used to replace the natural film and as medicinal preparation in some ocular pathologies such as the case of dry eye syndrome.

This invention is applicable to the areas of pharmacy and medicine.

### STATE OF THE ART

The ocular surface is known to be formed by the conjunctival epithelium, the accessory lacrimal glands and the meibomian glands. Said surface is coated by a continuous film, with a thickness of approximately 10 µm, called precorneal film or tear film. Until a few years ago, the theoretical structure, generally accepted, included three types of components (lipid, serum-aqueous, mucinous) distributed in three layers: lipid, aqueous and mucinous (Ibrahim H, Buri P, Gurny R. Pharm Acta Helv 1988, 63: 146-53).

Recent studies consider that the precorneal film is a structure formed by the aqueous-protein and mucinous components combined to form a hydrated gel. In turn, this gel would be protected by a film of lipid character, whose components would be mainly produced by the meibomian glands and whose function would be to prevent the evaporation of the tear and improve the stability of the tear film (Pflugfelder SC, Solomon A, Stern ME. Cornea 2000; 19 (5): 644-649. McCulley JP, Shine W. Tr Am Ophth Soc 1997; 95: 79-93).

In accordance with the proposed model, the precorneal film would consist of two phases:
- Hydrophilic polar phase, in contact with the aqueous-mucinous layer which is composed of phospholipids, sphingomyelin, ceramides and cerebrosides.
- Hydrophobic non-polar phase in contact with the atmosphere and composed of non-polar lipids such as wax esters, cholesterol esters, triglycerides, free fatty acids and hydrocarbons.

The fraction of phospholipids represents approximately between 1-5% of the total of lipid secretion, the greatest concentration being phosphatidylcholine (PC) with a percentage close to 40% of the total phospholipids. Other phospholipids, such as phosphatidylethanolamine appear in a percentage of 18%, the remainder (a total of 10) being found in a range between 3 and 9%. Probably, this fraction produces a reduction in the surface tension of the aqueous phase, facilitating the extensibility of the precorneal film during the blinking movement.

The usual treatment of dry eye consists of relieving the symptoms by applying tear replacements topically. The typical composition of these preparations includes polymeric solutions such as that included in US patent 4,973,580 (Babiole) in which the ophthalmic formulation includes hyaluronic acid using hydrogen peroxide as preservative. Formulations are also disclosed wherein components similar to tear film are provided such as hypotonic lecithin solutions including viscosity agents derived from cellulose as appears in the US patent no. 4,421,748 (Trager). The use of phospholipids for the treatment of dry eye appear in various patents. Emulsion-type systems including positively-charged phospholipids such as those described in the following are disclosed: US patent 4,914,088 (1990) (Korb:); 5,278,151 (1994) (Korb:); 5,371,108 (1994) (Korb:); 5,294,607 (1994) (Korb:). Likewise, positively-charged liposomes are disclosed (US patent no. 4,804,539 (Guo) (1989) and US patent no. 4,818,537 (Guo) wherein positively-charged liposomes are used which are suspended in aqueous solutions containing high-viscosity polymers such as hydroxyethyl cellulose, methylcellulose, hydroxypropyl cellulose and vinyl derivatives such as polyvinylpyrrolidone, polyvinyl alcohol and their mixtures. They also include emulsions containing phospholipids, non-polar oils and emulsifiers such as US patent no. 6,656,460 (Benita).
The patent application US5064655 refers to high viscosity liposome gel compositions, which, due to the viscous form persist at the site of application, especially mucosal issue. The composition includes a suspension of charged liposomes in a low-conductivity aqueous suspension medium which has a selected pH between 3,5 and 10,5.
The patent application WO2005/084635 refers to A pharmaceutical preparation suitable for use in the eye, which comprises: (i) a pharmaceutically acceptable carrier suitable for use in the eye; (ii) one or more ingredients selected from factors and agents that promote any one or more of survival, health, cell attachment and normal differentiation of ocular surface epithelial cells and optionally factors and agents to prevent squamous metaplasia; (iii) one or more agents capable of altering the fluid properties of a tear film including at least one agent capable of establishing and/or maintaining a stable tear film and optionally one or more agents selected from ophthalmological lubricating agents, viscosity enhancing agents and agents capable of reducing tear film evaporation.

In none of these patents does there appear the use of neutral or negatively-charged liposomes that are destabilized at the temperature of the precorneal film nor are they associated with mucin or with mucoadhesive substances or similar to mucin or mucomimetic as is the case of the invention described below.

### DESCRIPTION OF THE INVENTION

The method object of the invention described herein relates to the preparation of a pharmaceutical form which acts as replacement of the precorneal film. The formulation as defined by the claims incorporates liposomal vesicles of phospholipids as hydrophilic polar phase and non-polar lipids, both vehiculized in aqueous solutions which contain mucin or substances with mucomimetic substances or mucoadhesive polymers. The most relevant advantages of this invention consist of the use of phosphatidylcholine whose transition temperature is lower than the temperature of the corneal surface and also incorporates mucoadhesive and/or mucomimetic polymers or substances (mucin or polymers such as hyaluronic acid, cellulose derivatives, chondroitin sulphate, chitosan, colominic acid, thiolic derivatives or other similar components).

The components of the formulation and specifically the phospholipids which compose the liposomes are going to permit the formation, on the corneal surface, after the destabilization of the liposomal vesicles, of a water soluble monomolecular film which acts by preventing the evaporation of the aqueous phase and, furthermore, the surface tension of the latter will decrease which favours its rapid extensibility. The liposomes are prepared with phosphatidylcholine obtained from soy lecithin as majority component, cholesterol and α-tocopherol. Phosphatidylcholine contains acyl residues of fatty acids having a transition temperature lower than the temperature of the corneal surface, which guarantees the rapid formation of the film on the aqueous phase, once applied to the corneal surface. Cholesterol, for its part, stabilizes this film on reducing the fluidity of the matrix formed by the polyunsaturated residues of phosphatidylcholine. Finally, α-tocopherol ensures the chemical stability of the double bonds avoiding possible peroxidation.

The liposomes are vehiculized in aqueous solution containing an isotonizing agent (trehalose, sodium chloride, glucose...) to achieve the suitable osmolarity according to its clinical use. The solutions can be isotonic, or hypotonic. Once formed, the liposomes are incorporated in - aqueous solutions which contain one or several substances or polymers with mucoadhesive or mucomimetic characteristics with the purpose of producing an increase in the permanence time of the formulation and of the components of the destabilizing components on the ocular surface. Thus, the maintenance of the new film is favoured once formed and the aqueous evaporation of the corneal surface is avoided. The concentrations of this last component will depend on the desired final viscosity in the formulation, of its interaction with the mucin, of its surface tension and of the expected rheological composition after its administration. Proteins are also included in the formulation in order to favour the stability of the film formed and improve its lubricating properties. These proteins are found in the natural tears and are α-macroglobulin, lysozyme, lipocalin and lactoferrin.

To this formulation it is possible to add different components, in their majority components of natural tear film, which improve the characteristics of the formation and permanence of the precorneal film and/or which act as re-ephithelizers, anti-inflammatory agents and antioxidants of the ocular surface, and/or favouring corneal and conjunctival epithelial differentiation. Within these substances we have:
- Mucoadhesive polymers such as hyaluronic acid, cellulose derivatives, chondroitin sulphate, chitosan, colominic acid, thiolic derivatives (or another similar component).
- Neutral lipids and low polarity lipids such as waxes, cholesterol esters, triglycerides, free fatty acids and hydrocarbons.
- Vitamin A.
- Sodium,potassium, calcium, chloride and bicarbonate ions.
- Vitamin C.
- Albumin or pre-albumin.
- Immunoglobin A (IGA)
- Epithelial growth factor (EGF).
- Beta transforming growth factor (THF-β).
- Acidic fibroblast growth factor (aFGF).
- Basic fibroblast growth factor (bFGF).
- Antiproteases such as macroglobulin.
- Neural factors such as substance P and insulinlike growth factor.
- Antibacterial agents such as Ig G, lysozyme and complement.
- Long-chain fatty acids such as gadoleic, palmitic, palmitoleic, stearic, oleic, linoleic, arachidic, linolenic, eicosenoic, lignoceric, lactic and myristic acid.
- Hydrophilic lipids such as phospholipids, sphingomyelin, ceramides and cerebrosides.

### EMBODIMENT OF THE INVENTION

The present invention, which relates to the formulation of liposomal vesicles in aqueous solutions with characteristics of tear film, is additionally illustrated by the following examples, which are not limitative of their scope, which is defined by the attached note of claims.

The liposomal vesicles object of the invention were performed according to the classic Bangham method. To do this phosphatidylcholine, cholesterol and α-tocopherol (in different proportions) were dissolved in chloroform producing a final end concentration of phosphatidylcholine of 8 mg/ml. Once this solution is saturated with nitrogen it was introduced in the volumetric flask of the rotovapor at a temperature of 30-35ºC with moderate vacuum. After evaporating the solvent, a fine lipid film was formed on the walls which was then hydrated. The hydration phase was carried out with an aqueous solution saturated with nitrogen which contained the isotonizing agent at a temperature of 37ºC, using glass pearls which due to shear produced the formation of multilamellar vesicles. The final concentration of phosphatidylcholine was adjusted in accordance with the volume of the isotonizing vehicle.

After two hours of rest and in the absence of light, sonication of the dispersion was performed maintaining the product temperature between 5 and 10ºC with crushed ice. The preparation finalized performing 5 runs of the dispersion through 0.8 µm filters.

The mucin or mucoadhesive and/or mucomimetic substance was adding on diluting the liposomes to the desired final concentration. The final concentrations of the liposomes in the polymeric solution may range from 1 mg/ml to 40 mg/ml.

The other possible components are added, in accordance with their physicochemical characteristics, with the isotonizing agent or with the mucomimetic substances.

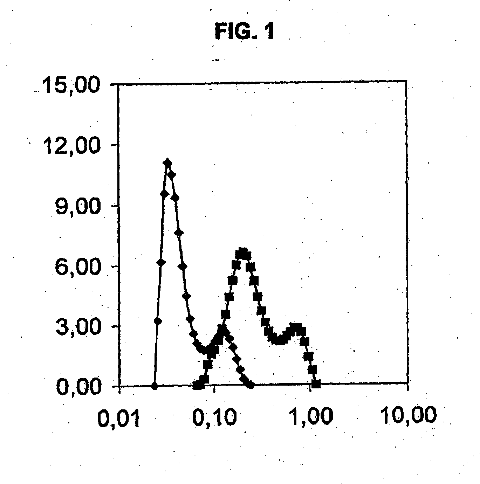

The *base* liposomes were prepared from phosphatidylcholine from soy, and cholesterol (8:1) and they were reconstituted with water and hypotonic solutions of sodium chloride. The influence of the sonication process on the end size of the vesicles was studied comparing the use of an ultrasound probe during 2.5 min and an ultrasound bath during 15 min (Fig. 1). The yield of the preparation process of the lipid vesicles, in both cases, was over 90%.

The dispersions of the liposomes in water at a PC concentration of 20 mg/ml had pH values between 6.9 and 7.2. The average particle diameters for the different batches prepared with ultrasound bath varied from 392 to 478 nm. The percentage of particles over 1 µm was, in all cases, under 2%.

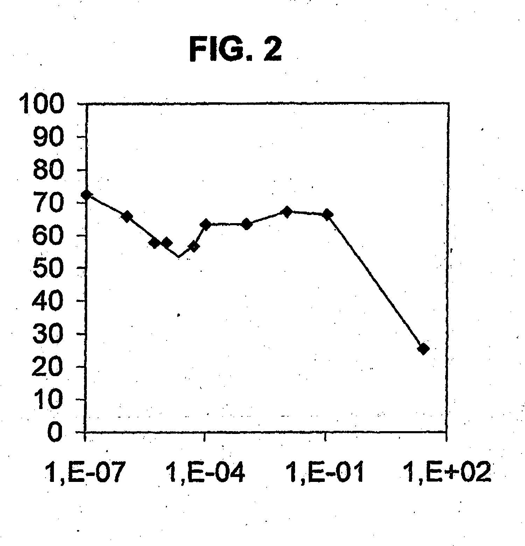

Measurements of surface tension was carried out with solutions of different liposome concentrations, obtaining the date in Figure 2.

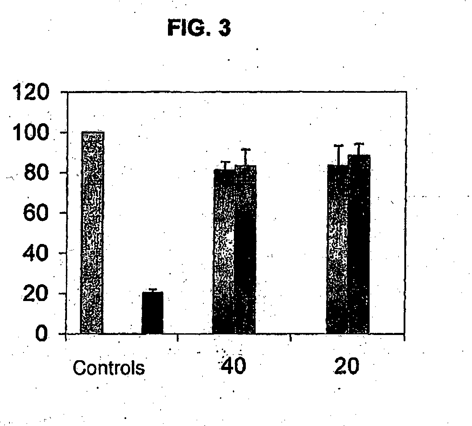

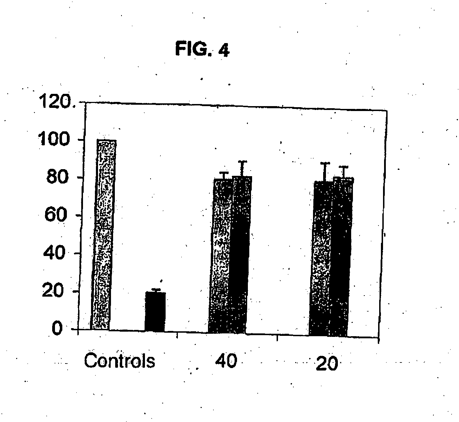

Cell viability tests were carried out with aqueous hypotonic solutions of *base* liposomes and *base* liposomes with vitamin E in cell cultures of macrophages. To study the cytotoxicity, the reduction technique was used, on a mitochondrial level, of the bromide salt of 3(4,5-dimethylthiazol-2-yl)-2,5-diphenyltetrazolium (MTT) to a coloured product (formazan) (Mossman T.J. Immum Methods 1983, 65:55-63). Peritoneal macrophages obtained from male Swiss mice were used. The cells were exposed to formulations which contained aqueous hypotonic solutions of *base* liposomes. As negative control, culture medium was used and as positive control 0.005% benzalkonium chloride. The solutions were incubated at 37ºC for 1 and 4 hours. The results obtained demonstrated an optimum tolerance for the *base* liposomes with and without vitamin E (Figures 3 and 4).

### BRIEF DESCRIPTION OF THE FIGURES

- Figure 1:: Influence of the sonication process on the final size of the vesicles comparing the use of an ultrasound probe during 2.5 minutes (-◆-) and an ultrasound bath during 15 minutes (-■-). The frequency of each class is represented, expressed in percentage, compared with the average size thereof in µm.
- Figure 2:: Surface tension of the aqueous dispersion of liposomes (mN/m) in accordance with its concentration. The concentration of the liposomes in the solution is expressed in phosphatidylcholine concentration (mM).
- Figure 3:: Cell viability (%) with aqueous hypotonic solutions of *base* liposomes with (■) and without (□) vitamin E incubated at 37ºC for 1 hour. Two concentrations of liposomes are studied (20 and 40 mg/ml) and two controls, one positive (0.005% benzalkonium chloride) and another negative (culture medium).
- Figure 4:: Cell viability (%) with aqueous hypotonic solutions of *base* liposomes with (■) and without (□) vitamin E incubated at 37ºC for 4 hours. Two concentrations of liposomes are studied (20 and 40 mg/ml) and two controls, one positive (0.005% benzalkonium chloride) and another negative (culture medium).

## Claims

1. Ophthalmic composition intended to act as replacement of the precorneal film, **characterized in that** it contains liposomal vesicles of phosphatidylcholine obtained from soy lecithin, cholesterol and α-tocopherol, vehiculized in aqueous solutions which contain mucin or substances with properties similar to mucin or mucoadhesives polymer, an isotonizing agent and proteins selected from α-macroglobulin, lysozyme, lipocalin and lactoferrin.

2. Ophthalmic composition according to claim 1, where the isotonizing agent is sodium chloride, trehalose or glucose.

3. Ophthalmic composition according to claim 1, where the mucoadhesive and/or mucomimetic substance is hyaluronic acid, cellulose derivatives, chondroitin sulphate, chitosan, colominic acid, thiolic derivatives.

4. Ophthalmic composition according to claim 1, which comprises vitamin A or vitamin C.

5. Ophthalmic composition, according to claim 1, which comprises sodium, potassium, calcium, chloride or bicarbonate ions.

6. Ophthalmic composition according to claim 1, which comprises albumin or pre-albumin.

7. Ophthalmic composition according to claim 1, which comprises immunoglobulin A (IgA).

8. Ophthalmic composition according to claim 1, which comprises growth factors such as epithelial growth factor (EGF), beta transforming growth factor (GF-β), acidic fibroblast growth factor (aFGF), basic fibroblast growth factor (bFGF).

9. Ophthalmic composition according to claim 1, which comprises antiproteases such as macroglobulin.

10. Ophthalmic composition according to claim 1, which comprises neural factors such as substance P or insulinlike growth factor.

11. Ophthalmic composition according to claim 1, which comprises antibacterial agents such as Immunoglobulin G (Ig G), lysozyme and complement.

12. Ophthalmic composition according to claim 1, which comprises long-chain fatty acids such as gadoleic, palmitic, palmitoleic, stearic, oleic, linoleic, arachidic, linolenic, eicosenoic, lignoceric, lactic and myristic acid.

13. Ophthalmic composition according to claim 1, which comprises hydrophilic lipids such as phospholipids, sphingomyelin, ceramides and cerebrosides.

14. Ophthalmic formulation of any of claims 1-13 for use in curing dry eye syndrome.

## Patentansprüche

1. Am Auge zu verwendende Zubereitung, die vorgesehen ist, als Ersatz für den präcornealen Film zu wirken, **dadurch gekennzeichnet, dass** sie liposomale Vesikeln aus Phosphatidylcholin, das aus Sojalecithin, Cholesterin und α-Tocopherol erhalten worden ist, enthält, die von wässrigen Lösungen getragen werden, die Mucin oder Substanzen mit Eigenschaften, die denjenigen des Mucins oder eines mukoadhäsiven Polymers ähnlich sind, ein isotonisierendes Mittel und Proteine, die aus α-Makroglobulin, Lysozym, Lipocalin und Lactoferrin ausgewählt sind, enthalten.

2. Am Auge zu verwendende Zubereitung nach Anspruch 1, wobei das isotonisierende Mittel Natriumchlorid, Trehalose oder Glucose ist.

3. Am Auge zu verwendende Zubereitung nach Anspruch 1, wobei die mukoadhäsive und/oder mukomimetische Substanz Hyaluronsäure, Cellulosederivate, Chondroitinsulfat, Chitosan, Colominsäure und Thiolderivate ist/sind.

4. Am Auge zu verwendende Zubereitung nach Anspruch 1, die Vitamin A oder C umfasst.

5. Am Auge zu verwendende Zubereitung nach Anspruch 1, die Natrium-, Kalium-, Calcium, Chlorid- oder Hydrogencarbonationen umfasst.

6. Am Auge zu verwendende Zubereitung nach Anspruch 1, die Albumin oder Präalbumin umfasst.

7. Am Auge zu verwendende Zubereitung nach Anspruch 1, die Immunglobulin A (IgA) umfasst.

8. Am Auge zu verwendende Zubereitung nach Anspruch 1, die Wachstumsfaktoren wie den epithelialen Wachstumsfaktor (EGF), β-transformierenden Wachstumsfaktor (TGF-β), sauren Fibroblastenwachstumsfaktor (aFGF) und basischen Fibroblastenwachstumsfaktor (bFGF) umfasst.

9. Am Auge zu verwendende Zubereitung nach Anspruch 1, die Antiproteasen wie Makroglobulin umfasst.

10. Am Auge zu verwendende Zubereitung nach Anspruch 1, die neurale Faktoren wie die Substanz P oder den insulinähnlichen Wachstumsfaktor umfasst.

11. Am Auge zu verwendende Zubereitung nach Anspruch 1, die antibakterielle Mittel wie Immunglobulin G (IgG), Lysozym und ein Komplement umfasst.

12. Am Auge zu verwendende Zubereitung nach Anspruch 1, die langkettige Fettsäuren wie Gadolein-, Palmitin-, Palmitolein-, Sterin-, Öl-, Linol-, Arachin-, Linolen-, Eicosen-, Lignocerin-, Milch- und Myristinsäure umfasst.

13. Am Auge zu verwendende Zubereitung nach Anspruch 1, die hydrophile Lipide wie Phospholipide, Sphingomyelin, Ceramide und Cerebroside umfasst.

14. Am Auge zu verwendende Zubereitung nach einem der Ansprüche 1 bis 13 zur Verwendung bei der Heilung des Trockenen-Auge-Syndroms.

## Revendications

1. Composition ophtalmique destinée à servir comme remplacement de film lacrymal, **caractérisée en ce que** elle contient des vésicules de liposome de phosphatidylcholine obtenues à partir de lécithine de soja, cholestérol et alpha-tocophérol, véhiculisées dans des solutions aqueuses qui contiennent de la mucine ou des substances ayant des propriétés similaires à la mucine ou des polymères muco-adhésifs, un agent isotonisant et des protéines choisies à partir de macroglobuline, lysozyme, lipocaline et lactoferrine.

2. Composition ophtalmique selon la revendication 1, où l'agent isotonisant est chlorure de sodium, tréhalose ou glucose.

3. Composition ophtalmique selon la revendication 1, où le muco-adhésif et/ou la substance muco-mimétique est l'acide hyaluronique, des dérivatifs de cellulose, le sulfate de chondroïtine, le chitosane, l'acide colominique, ou des dérivatifs thiolique.

4. Composition ophtalmique selon la revendication 1, qui comprend de la vitamine A ou de la vitamine C.

5. Composition ophtalmique selon la revendication 1, qui comprend du des ions de sodium, potassium, calcium, chlorure ou de bicarbonate.

6. Composition ophtalmique selon la revendication 1, qui comprend de l'albumine ou pré-albumine.

7. Composition ophtalmique selon la revendication 1, qui comprend de l'immunoglobuline A (IgA).

8. Composition ophtalmique selon la revendication 1, qui comprend des facteurs de croissance comme le facteur de croissance épithéliale (EGF), le facteur de croissance transformant beta (GF-β), le facteur de croissance acide de fibroblaste (aFGF), le facteur de croissance basique de fibroblaste (bFGF).

9. Composition ophtalmique selon la revendication 1, qui comprend des anti-protéases comme la macroglobuline.

10. Composition ophtalmique selon la revendication 1, qui comprend de facteurs neuraux comme la substance P ou le facteur de croissance analogue à l'insuline.

11. Composition ophtalmique selon la revendication 1, qui comprend des agents anti-bactériels comme l'immunoglobuline G (Ig G), lysozyme et complément.

12. Composition ophtalmique selon la revendication 1, qui comprend des acides gras à chaine longue comme l'acide gadoléique, palmitique, palmitoléique, stéarique, oléique, linoléique, arachidique, linolénique, eicosénoïque, lignocérique, lactique, et myristique.

13. Composition ophtalmique selon la revendication 1, qui comprend des lipides hydrophliques comme les phospholipides, sphingomyéline, céramides et cérébrosides.

14. Formulation ophtalmique selon l'une quelconque de revendications 1 à 13 pour l'utilisation dans la guérison du syndrome de l'oeil sec.
